# EUROPEAN PATENT APPLICATION

(11) **EP 1 275 369 A1**
(43) Date of publication of application: **15.01.2003**
(21) Application number: 01202646.4
(22) Date of filing: 10.07.2001
(51) Int. Cl.: A61K 7/48, A61K 7/00

(54) **Folic acid containing personal care product**

(71) Applicant: Goldschmidt AG, 45127 Essen (DE)
(72) Inventor: Brand-Garnys, E. E., 2914 AD Nieuwerkerk a/d Ijssel (NL); Heijmeriks, A. C., 3062 JL Rotterdam (NL)
(74) Representative: Visser-Luirink, Gesina, Dr.

(57) **Abstract**

A personal care product consisting of an oil-in-water emulsion incorporating a high concentration of folic acid dissolved in a liquid crystalline matrix was found to reduce the tactility and visibility of certain chronic superficial skin blemishes and topic hyperkeratotic spots in particular.

## Description

### FIELD OF THE INVENTION

The invention concerns a personal care product and the use of said personal care product in reducing the tactility and visibility of certain superficial skin blemishes.

### BACKGROUND OF THE INVENTION

Folate deficiency has been reported to be a common vitamin deficiency in the Western world and especially with juveniles and elderly. Deficiency of folate in the diet is likely to result in a reduction in the capacity to synthesize DNA and in maintaining the usual rate of cell division. Inadequate folate intake during pregnancy has been linked with neural tube effects in newborn. More recently folate has also been implicated in the development of cancers and cardiovascular diseases.
It is well established that folate supplies one-carbon units required for RNA and DNA synthesis as well as for numerous methylation reactions that take place in living organisms. Folate deficiency has been shown to result in chromosomal damage, DNA hypomethylation and misincorporation of uracil into DNA.

In view of its important role in the human metabolism and the likelihood of inadequate supply, the US Food and Nutrition Board has suggested to increase the recommended folate intake for adults to 400 micrograms per day from the previous 200 microgram per day.
The reason for the widespread occurrence of folate deficiency is the absence in humans of enzymes to synthesize folate from the basic building blocks so that there is a total dependence upon dietary intake. Although fruit and vegetables contain relatively high levels of folate, these levels are low in relation to the desired daily requirements. Moreover, naturally occurring folates are labile compounds that are easily destroyed during food storage and food processing, for example during cooking of the vegetables. Folates are soluble in water and consequently processing of vegetables results in loss of folates as the result of leaching (J Sci Food Agric 80:795-824 (2000). In this way at least a substantial portion of the folates is discarded together with the water used for processing.

The intake of sufficiently high levels of folates depends upon the consumption of relatively large portions of fresh fruits and vegetables. However, in view of the dietary behaviour of many individuals this is not very likely and suppletion of folates to the diet is strongly recommended. This is usually done by separate consumptions of synthetic folic acid. Folate depletion is known to be restored within 3 weeks following a diet high in natural folates. Any dietary surplus of natural folates or synthetic folic acid consumed is eliminated via the usual catabolic pathways and the degradation products are ultimately excreted. Several scientific reviews have concluded that folic acid is safe for oral consumption under most circumstances.

The chemistry of folates makes this vitamin one of the most vulnerable to losses, not only as the result of its solubility in water but also as the result of exposure to oxygen. Preliminary evidence to suggest that folates can also be destroyed upon exposure to UV light also exists. For example, human plasma exposed to stimulated strong sunlight in vitro shows a rapid loss of folate. Furthermore, light skinned patients exposed to long wavelength ultraviolet light were found to have abnormally low serum folate levels (Science 1978; 201: 625-626). Hence the exposure to sunlight and UV irradiation in particular may be expected to lower folate levels in the superficial layers of the skin with a concomitant disabling effect of the skin's DNA repair system. Difficulties of the DNA repair system of healthy subjects to cope with the avalanche of DNA damage caused by high doses of UB-B was recently illustrated by the beneficial effect of a topical addition of the DNA repair enzyme photolyase (PNAS 2000: 97: 1790-1795). One might conclude that under certain oppressive conditions the existing defense system of the skin is hardly adequate which makes an impaired DNA repair system, for example as the result of inadequate folate levels, highly undesirable. Despite the vast information that is available on the fate of orally taken folate, there are no scientific reports describing the effects of topically applied folate or folic acid. It is even unknown if topically applied folates or folic acid can penetrate the skin to add to the subcutaneous folate levels.

Folate or its synthetic precursor folic acid are also known as vitamin B9 and these compounds thus belong to the group of B-vitamins. Folate (i.e. vitamin B9) plays an important role in the one- carbon mechanism required for DNA and RNA synthesis (R.A. Jacob in Advances in Nutrition and Cancer 2; edited by Zappia et al., Kluwer Academic/ Plenum Publishers, New York, 1999). In the reaction equilibria of folate's enzymatic pathways, other members of the group of B-vitamins such as vitamin B12 (cyanobalamin) and vitamin B6 (pyridoxine) play important roles as well.
Orally taken folic acid is first converted into a metabolically inactive form until it is demethylated to tetrahydrofolate. In the latter step vitamin B12 plays a crucial role and like folate, vitamin B12 deficiencies have also been linked with inadequate DNA metabolism and increased chromosome damages (J Sci Food Agric 80: 795-824 (2000)). Vitamin B6 is involved as a cofactor in the enzymatic transformation of homocysteine into cysteine and thus vitamin B6 shortages can also be expected to influence DNA and RNA synthesis (J Sci Food Agric 80: 795-824 (2000)).
Although less directly involved than the vitamins B6 and B12, vitamin B3 (niacin) is also known to affect the metabolism of DNA. Very recently vitamin B3 could be shown to provide the ADP-ribose units for proteins modulating DNA replication and repair in humans (Jacob, R.A. in Advances in Nutrition and Cancer 2, edited by Xappia et al, Kluwer Academic/ Plenum Publishers, New York, 1999).
Other B-vitamins such as vitamin B5 (panthothenic acid) and vitamin B8 (biotin or vitamin H) have no known direct effects on the metabolism of DNA, but exhibit several beneficial topical effects if incorporated in personal care products. For example, proven benefits of vitamin B5 (or its precursor panthenol) include skin moisturisation and anti-inflammatory effects if incorporated in skin care products in levels of 0.5% or higher. Other benefits have been reported for hair care and nail care products (G. Erlemann and R. Merkle in Seifen, Ole, Fette, Wachse, vol 117, no10 (1991)). Vitamin B8 is associated with the process of cell development and lipid metabolism. In personal care products it is typically added to promote the condition of hair and nails so that a beneficial effect on keratinocyte formation is very likely.
In addition to their effects on the metabolisme of nucleic acids and their stimulatory role on cell formation, it is also known that topically applied vitamins are capable of normalising the microbiological flora of the skin. For example in relation to the incidence of certain desirable microbiological strains. Important to note is that the naturally occurring folates as well as folic acid and the other above mentioned B-vitamins are all listed as water-soluble compounds (Biotechnology of Vitamins, Pigments and Growth Factors; E.J. Vandamme ed; ISBN 1-85166-325-8). This water-solubility has important consequences for the preparation of personal care products incorporating these vitamins.

Technically speaking many personal care products are multi-phase systems consisting of at least two mutually immiscible liquids (such as water and oil) plus a single or a mixture of amphiphilic product(s). At least one of the immiscible liquids is present in a finely divided state (the dispersed phase) in the other liquid (the continuous phase). The amphiphilic products are usually referred to as "emulsifiers".
Multi-phase systems are usually stabilised by self-aggregation of the amphiphilic products. This self-aggregation is characterised by a structured agglomeration of the amphiphilic products to such an extent that hydrophilic-hydrophobic repulsion is minimised. Self-aggregation of these amphiphilic products may occur in three forms, i.e. in spherical, tubular or plate-like structures. The prevailing form depends on the geometry of the amphiphilic products used (J.N.Israelachvili, D.J.Mitchell, B.W.Ninham, J.Chem.Soc., Faraday Trans.2, 72, 1525, (1976); D.R.J.Clymans, H.M.Brand, Cosmetics and Toiletries Manufacturing Worldwide, 1995, "Polymeric Emulsifiers; an Alternative to Traditional Emulsifiers based on Stability of Multi-Phase Systems". ISBN 0.9519830.4.0).
Spherical and tubular structures obtained through self-aggregation are usually referred to as "micelles". Such micelles are frequently encountered in wash-active products such as shampoos, detergents, etc. and are not relevant within the scope of the present invention.
Plate-like self-assemblies of amphiphilic products are preferred constituents of emulsions that are frequently encountered in personal care products. Such plate-like structures are characterised by a double layer of head-tail/ tail-head oriented amphiphilic molecules that physically separate the dispersed droplets and thus stabilise the emulsion. These double layered plate-like structures are identified as liquid crystalline entities that exhibit anisotropy. Anisotropy of this liquid crystalline double layer can be demonstrated by optical microscopy using polarised light and a magnification of 200-400times. In case a liquid crystalline structure is present in emulsions this becomes manifest by the presence of "Maltese crosses" or "oily streaks". Quantification of the liquid crystalline double layer formed is possible using advanced methods but is usually done by visually estimating the concentration of the Maltese crosses or oily streaks through the microscope (K. Shinoda *et al*, J.Colloid Interface Sci., 80, 304 (1981); H. Kunieda and S.E. Friberg, Bull. Chem. Soc. Japan, 54, 1010 (1981)).

The mechanical strength of the liquid crystalline double layer alone is usually insufficient to assure long term stability of the emulsion formed (Tharwat Tadros in "Emulsions: Technology, Structure, Ingredients, Formulations", Verlag fur Chemische Industrie H. Ziolkowsky GmbH, ISBN 3-87846-196-8). To increase the mechanical strength the liquid crystalline double layer is usually blanketed with a suitable rheological additive. The interaction by and between the liquid crystalline double layer and a suitable rheological additive is based on Vanderwaals interactions. Suitable rheological additives are usually polysaccharides such as tree and shrub exudates like gum arabic, fermentation products such as xanthan gum, seed extracts such as guar gum, seaweed extracts such as alginates, plant derived materials such as pectins, starches, cellulose or cellulose derivatives such as hydroxyethylcellulose. Also synthetic rheological additives such acrylic homo- and copolymers, and modified clays such as smectites, hectorites, bentonites, hydrated silica and fumed silica are useful.

Personal care products and especially skin care products are usually based upon very stable emulsions of an oil phase in a continuous water phase. Such so-called Oil-in-Water (O/W) emulsions are typically prepared by dissolving the rheological additive as well as the other water soluble components, for example water-soluble vitamins belonging to the group of B-vitamins, in the water phase. The suitable emulsifiers are dissolved in the oil phase and then the oil phase is added to the water phase (usually at elevated temperatures) using a suitable homogeniser such as a rotor-stator mixer at high velocity. This approach yields a stable emulsion which guarantees a good bioavailability of the various water soluble vitamins added. We have found that this approach is not suited for incorporating folic into an emulsion because in the concentrations as required, the folic acid cannot be incorporated in a stable and bio-available form.

The epidermal skin can reveal several types of blemishes or imperfections. Here we define such blemishes or imperfections as being epidermal, superficial, localised, non-acute, non-infectious and visible, tactile or both. These blemishes can include small spots with a deviating colour such as caused by irregular pigmentation or verruceous growth or erythrosis as well as epidermal lesions known as maculae, naevi, ulcers, scales and local hyperkeratosis. Blemishes may also occur on the scalp. However, blemishes occur, or are thought to occur, most frequently on the exposed, and thus visible, parts of the skin such as the face, arms and legs. Because of the psychological connection with an aged skin, these blemishes are considered non-esthetic imperfections. Therefore, there is a need to alleviate the visibility and tactility of such imperfections.

### SUMMARY OF THE INVENTION

We have found that folic acid can be substantially dissolved in an oil-in-water emulsion, preferably an oil-in-water emulsion characterised by the presence of large numbers of liquid crystalline entities.
Surprisingly these emulsions and personal care products incorporating these emulsions and optionally at least one vitamin belonging to the group of B-vitamins, are useful to treat tactility or visibility of superficial skin blemishes or to treat tactility or visibility of hyperkeratotic skin.

### DETAILED DESCRIPTION OF THE INVENTION

Personal care products are products that are applied to skin, hair, nails and mucous membranes to care, cleanse or decorate and to maintain and improve the quality of skin, hair, nails and mucous membranes. Many personal care products are based upon emulsions. Emulsions are defined as systems of at least two mutually immiscible liquids whereby at least one of these liquids is present in a finely divided state in at least one of the other liquids. In emulsions either water or oil can be the continuous phase depending on the emulsifier or emulsifiers used. Within the scope of this invention Oil-in-Water (O/W) emulsions are considered in which the water phase is the continuous phase and the oil phase the dispersed phase.

It is known that the optimal formation of a liquid crystalline structure by emulsifiers in O/W emulsions is coincident with an HLB value of 10 of the emulsifier or emulsifier combination (J.N.Israelachvili, D.J.Mitchell, B.W.Ninham, J.Chem.Soc., Faraday Trans.2, 72, 1525, (1976)). In the case of using combinations of emulsifiers current practice is to incorporate the lipophilic emulsifier (HLB < 10) in the oil phase of the emulsions and the hydrophilic emulsifier (HLB > 10) in the water phase of the emulsion. Examples of emulsifiers suitable for the preparation of liquid crystalline structures include ethoxylated/propoxylated fatty alcohols, ethers of fatty alcohols and polyhydric alcohols such as polyglycerol-x whereby x=2-20, carbohydrates such as sorbitol, fructose, glucose, methylglucose, polyglucose (alkyl polyglucosides), esters of fatty acids with suitable polyhydric alcohols such as glycerol, trimethylolpropane, pentaerythritol, polyethylene glycol (n=1-200), polypropylene glycol (n=1-50), copolymers of ethylene oxide, propylene oxide, tetrahydrofuran, and combinations thereof. Also some electrically charged emulsifiers form a liquid crystalline bilayer such as lecithins and kephalins (1,2-diacyl glycerophosphates, as a condensate with serine, inositol, choline or monoethanolamine) or other phospholipids such as sphingolipids or glycolipids.

It is known that particular emulsifiers/emulsifier combinations allow the incorporation of foreign chemical compounds within the liquid crystalline double layer. To guarantee adequate bio-availability, these foreign chemical compounds should preferably be dissolved in the liquid crystalline double layer. For example, it has been shown that particular corticosteroids can be dissolved in a double layer formed by lecithin. In this way it was possible to combine a good bio-availability with a sustained release of these corticosteroids from the emulsion.
However, it is impossible to predict which chemical compounds can be dissolved in the liquid crystalline double layer. Furthermore it is not possible to predict which emulsifier or combination of emulsifiers are required to enable the dissolution of particular chemical compounds in the liquid crystalline double layer of an O/W emulsion. Generally speaking it could be expected that the principle of "like-dissolves-like" would apply. So in case the structure of the bilayer would be of the type H-L...L-H ("H" representing a hydrophilic moiety and "L" an lipophilic moiety) it would be expected that lipophilic substances would dissolve in the core of the bilayer. However, in some cases the contrary has been observed : hydrophilic substances with a conflicting dielectricum may molecularly dissolve in particular double layers while substances with a non-conflicting dielectricum may not. In addition to these polarities of the liquid crystalline bilayer and the substrate, a number of other parameters have been taken into consideration such as the composition of the continuous phase, the identity of the dispersed phase, the phase ratio, the presence of co-surfactants etc. Unfortunately no correlation has been shown to have predictive value relative to the possibility of dissolution of foreign substances in liquid crystalline double layers (S.E. Friberg and P. Bothorel; "Microemulsions: Structure and Dynamics"; CRC Press,1987,1995, ISBN 0-8493-6598-8). In fact it is presently not known which parameters determine the solubility of foreign chemical compounds in liquid crystalline bilayers of O/W emulsions.

Preferred emulsifiers that allow the production of O/W emulsions in which folic acid can be dissolved in the liquid crystalline double layer comprise alkyl glucosides such lauryl, mirystyl, cetyl, cetearyl, stearyl, arachidyl and behenyl glucoside; sucrose esters such as sucrose cocoate, stearate and distearate; sorbitan esters such as sorbitan palmitate and stearate; fatty alcohol ethoxylates such as ethoxylated cetyl alcohol and stearyl alcohol; polyglyceryl esters and ethers of common fatty acids and fatty alcohols. Most preferred emulsifiers enabling the formation of emulsions containing dissolved folic acid are alkyl glucosides. We have found that using these preferred emulsifiers the folic acid added can be substantially dissolved in the liquid crystalline layers of the emulsion. Substantially dissolved meaning that less than 10 %, preferably less than 1% of folic acid added is visible as crystallites in the final emulsion as judged by optical microscopy (magnification 200-400 x) in combination with polarised light.
Successful formation of the emulsion is not only characterised by the substantial dissolution of folic acid, but also by considerable anisotropy. Visual inspection of a successfully formed emulsion through the microscope using polarised light will reveal large numbers of liquid crystalline entities such as Maltese crosses and/or oily streaks. Large numbers meaning that 5- 10000, preferably 50-1000 Maltese crosses and/or oily streaks are visible in a single microscope field looking at the emulsion with a magnification of 200x .
Knowing that folic acid can be substantially dissolved into liquid crystalline double layers using the preferred emulsifiers, the skilled person will appreciate that other emulsifiers can also be used provided that the geometrical configuration of the double layer structure formed is suitable. O/W emulsions according to the invention may incorporate folic acid in quantities that range from 0.1 microgram/g emulsion to 5 milligram/g emulsion.

To achieve adequate shelf stability of the O/W emulsions containing substantially dissolved folic acid, it is preferred that the liquid crystalline double layers holding the folic acid and formed by the selected emulsifiers are reinforced by a suitable rheological additive. Suitable rheological additives include polysaccharides such as tree and shrub exudates (karaya gum, tragacanth gum, gum arabic, gum ghatti, etc), fermentation products such as xanthan gum, seed extracts such as guar gum (and derivatives), locust bean gum, quince seed gum, psyllium seed gum, tamarind seed gum, seaweed extracts such as alginates (and esters thereof) and carrageenan (various forms), agar-agar, pectins, starches, cellulose derivatives such as methylcellulose, hydroxyethylcellulose, hydroxypropyl methylcellulose. Also synthetic rheological additives such acrylic homo- and copolymers, and modified clays such as smectites, hectorites, bentonites, hydrated silica and fumed silica are useful.
Preferred rheological additives to stabilise O/W emulsions incorporating substantially dissolved folic acid are carbomers, xanthan gum, locust bean gum, hydroxyethylcellulose, hydroxypropylcellulose and hydroxypropyl methylcellulose. The required concentration of these rheological additives may vary depending on the nature of the rheological additive chosen but typically ranges from 0.01-2.00 %.

Thus a stable O/W emulsion exhibiting a high bioavailability of folic acid can be prepared from water, an oil phase such as cyclopentasiloxane( 1-40%), a selected emulsifier such as cetearyl glucoside/cetearyl alcohol (0,5-15%, preferably 2,5%) and folic acid (0.01-0.5%, preferably 0.1-0.3% w/w). Optionally other components can be added to stabilise the emulsion or to improve the performance of the final personal care product, for example a suitable rheological additive such as xanthan gum (0,05-0,8 %), lipophilic compounds intended for softening and/or smoothing of the skin such as mineral hydrocarbons, fatty acid esters, polydimethylsiloxanes, vegetable oils, ethoxylated or propoxylated fatty alcohols, benzoic acid esters etc (0.1-20 %), humectants such as alcohols and/or polyols such as glycols, glycerol, sorbitol, methylglucose, polyglycerol or polysaccharides and ethoxylates/ propoxylates thereof (0.05-20%), cosmetic actives such as vitamins, enzymes or botanical extracts ( 0.001 - 10%) and cosmetically acceptable preservatives such as parabene cocktails supported by co-preservatives (0.1-1.0 %). The emulsion can be made in a temperature range of 30-90°C, preferably 55-70°C, in a pH range of 4.0-9.0, preferably 5.0-6.5.

If properly made, the emulsion obtained will contain large numbers of liquid crystalline entities as demonstrated by the large numbers of Maltese crosses or oily streaks that are visible through the microscope using polarised light. If properly incorporated into the liquid crystalline matrix of the emulsion, folic acid will be substantially dissolved as judged by optical microscopy with a magnification of 200-400 times.

To test the effects of O/W emulsions according to the invention, samples of the emulsions have been applied daily and for extended periods of time on the skin of a group of individuals. Quite unexpectedly a number of test persons reported significant reductions in the visibility and tactility of a variety of superficial skin blemishes. Most notable were the effects on minor blemishes involving irregular pigmentation or erythrosis as well as epidermal lesions known as maculae, naevi, ulcers, scales and hyperkeratotic spots that can be seen on the skin of individuals with a history of overexposure to mitogens such as UV irradiation or chemical irritants. In some cases verruceous tissue was also found to decline as the result of the regular exposure to the folic acid containing emulsion according to the invention.

To increase the cosmetic efficacy of the folic acid containing emulsions according to the invention, additional vitamins that act in synergy with folic acid can be added to obtain personal care products with a broad spectrum of activity.

Preferred synergistically working vitamins are the water-soluble vitamins of the B-group which include vitamin B12 (cyanobalamin), vitamin B6 (pyridoxol), vitamin B3 (niacin), vitamin B5 (panthothenic acid or its precursor panthenol) and vitamin B8 (biotin). Most preferred are vitamins B3, B6 and B12. Suitable concentrations of these vitamins in the end product range from 1-100 mg/g product of vitamin B3, from 0.2-20 mg/g of vitamin B6, from 0.2-20 microgram/g of vitamin B12, from 1-100 mg/g of vitamin B5, from 0.03-3 mg/g of vitamin B8. In contrast with folic acid, there is no need to incorporate these B-vitamins into the liquid crystalline double layer, i.e. these vitamins can be added to the formulation directly with the aqueous phase in the manufacturing process and thus separately from the folic acid. These vitamins can be added as single additions or in combinations incorporating various vitamins.

The following Examples will illustrate the invention.

### EXAMPLE 1

The following formula illustrates the composition and production of a cosmetic formulation in which folic acid has been incorporated into the liquid crystalline matrix of an O/W emulsion.

### COMPOSITION:

| Phase-A: | |
|---|---|
| DEMINERALISED WATER | 41.50 % |
| INCI: Aqua | |
| ARLATONE® 2121 | 5.00 % |
| INCI: Sorbitan Stearate, Sucrose Cocoate | |
| PROPYLENE GLYCOL | 2.50 % |
| INCI: Propylene Glycol | |
| GLUCAM® E20 | 1.55 % |
| INCI: Methyl Gluceth-20 | |
| PHENONIP® | 0.70 % |
| INCI: Phenoxyethanol, Methylparaben, Ethylparaben, Butylparaben, Propylparaben, Isobutylparaben | |
| NIPAGUARD DCB® | 0.05 % |
| INCI: Phenoxyethanol, Methyldibromo Glutaronitrile | |
| | |

| Phase-B: | |
|---|---|
| DEMINERALISED WATER | 15.00 % |
| INCI: Aqua | |
| RHODICARE® D | 0.20 % |
| INCI: Xanthan Gum | |
| | |

| Phase-C: | |
|---|---|
| DEMINERALISED WATER | 10.00 % |
| INCI: Aqua | |
| CITRIC ACID, 10% SOLUTION | 0.55 % |
| INCI: Aqua, Citric Acid | |
| FOLIC ACID | 0.15 % |
| INCI: Folic Acid | |
| | |

| Phase-D: | |
|---|---|
| MYRITOL® 318 | 8.00 % |
| INCI: Caprylic/Capric Triglyceride | |
| DOW CORNING® 345 | 6.00 % |
| INCI: Cyclomethicone | |
| ARLAMOL® E | 3.50 % |
| INCI: PPG-15 Stearyl Ether | |
| LANETTE® 22 | 2.00 % |
| INCI: Behenyl Alcohol | |
| ARLACEL® 165 | 1.80 % |
| INCI: Glyceryl Stearate, PEG-100 Stearate | |
| ESTOL® E04BW 3752 | 1.50 % |
| INCI: PEG-8 Beeswax | |

Arlatone, Arlamol and Estol are registered trademarks of Uniqema; Glucam is a registered trademark of Union Carbide c/o Amerchol; Phenonip & Nipaguard are registered trademarks of NIPA Laboratories; Rhodicare is a registered trademark of Rhodia; Myritol and Lanette are registered trademarks of Cognis; Dow Corning is a registered trademark of Dow Chemical. Folic acid was obtained from E.Merck, Darmstadt, Germany.

### PROCEDURE:

1. To prepare phase A, ARLATONE 2121 is mixed with water and heated to 85-90°C. A gel will be formed. The gel is cooled to 60°C. Propylene glycol and Glucam E20 are added and mixed until homogeneous. Phenonip and Nipaguard DCB are added.
2. To prepare phase B, Rhodicare D is dispersed in water and heated to 60°C while stirring gently.
3. Phase B then added to phase A and stirred until homogeneous. The temperature is maintained at 60°C.
4. Phase C is prepared by heating to 60°C while mixing gently. Phase C is then added to phase {A+B} while stirring gently. The gel is monitored by microscope (100-400times magnification) for the absence of crystallites of folic acid.
5. Phase D is prepared by heating and mixing to 60°C. Phase D is then added to phase {A+B+C} without mixing. The mixture is homogenized during 20 seconds and the emulsion is subsequently stirred until ambient temperature has been reached.

### PRODUCT EVALUATION:

After cooling down the emulsion obtained has a viscosity of 12.500 cPs Brookfield viscosity (S5; 20 rpm; 20°C) and a pH of 6,15. The emulsion is evaluated microscopically for the absence of crystallites and the presence of "Maltese crosses". If crystallites are present, step 4 of the procedure has not been performed up to the requirements. If no or little Maltese crosses can be observed, the liquid crystalline phase has not been formed.

### EXAMPLE 2

The following formula illustrates the composition and production of a cosmetic formulation in which folic acid has been incorporated into the liquid crystalline matrix and vitamin B8 and provitamin B5 in the aqueous phase of an O/W emulsion.

### COMPOSITION:

| Phase-A: | |
|---|---|
| DEMINERALISED WATER | 18.30 % |
| INCI: Aqua | |
| BRIJ® 721 | 2.50 % |
| INCI: Steareth-21 | |
| PROPYLENE GLYCOL | 2.50 % |
| INCI: Propylene Glycol | |
| GLUCAM® E20 | 1.55 % |
| INCI: Methyl Gluceth-20 | |
| d,1-PANTHENOL (PROVITAMIN B5) | 1.00 % |
| INCI: Panthenol | |
| PHENONIP® | 0.70 % |
| INCI: Phenoxyethanol, Methylparaben, Ethylparaben, Butylparaben, Propylparaben, Isobutylparaben | |
| NIPAGUARD DCB® | 0.05 % |
| INCI: Phenoxyethanol, Methyldibromo Glutaronitrile | |
| | |

| Phase-B: | |
|---|---|
| DEMINERALISED WATER | 30.00 % |
| INCI: Aqua | |
| CARBOPOL® ETD2020 | 0.20 % |
| INCI: Acrylates/C₁₀₋₃₀ Alkyl Acrylates Crosspolymer | |
| CARBOPOL® ETD2050 | 0.20 % |
| INCI: Carbomer | |
| RHODICARE® D | 0.10 % |
| INCI: Xanthan Gum | |
| | |

| Phase-C: | |
|---|---|
| DOW CORNING® 345 | 10.00 % |
| INCI: Cyclomethicone | |
| MYRITOL® 318 | 5.00 % |
| INCI: Caprylic/Capric Triglyceride | |
| ARLAMOL® E | 4.00 % |
| INCI: PPG-15 Stearyl Ether | |
| BRIJ® 72 | 2.50 % |
| INCI: Steareth-2 | |
| LANETTE® 18 | 2.00% |
| INCI: Stearyl Alcohol | |
| ESTOL® E04BW 3752 | 2.00 % |
| INCI: PEG-8 Beeswax | |
| ARLACEL® 165 | 1.80 % |
| INCI: Glyceryl Stearate, PEG-100 Stearate | |
| | |

| Phase-D: | |
|---|---|
| DEMINERALISED WATER | 10.00 % |
| INCI: Aqua | |
| CITRIC ACID, 10% SOLUTION | 0.55 % |
| INCI: Aqua, Citric Acid | |
| FOLIC ACID | 0.15% |
| INCI: Folic Acid | |

| Phase-E: | |
|---|---|
| SODIUM HYDROXIDE SOLUTION, 18% | 0.10 % |
| INCI: Aqua, Sodium Hydroxyde | |
| | |

| Phase-F: | |
|---|---|
| PRICERINE 9099 | 5.00 % |
| INCI: Aqua, Sodium Hydroxyde | |
| VITAMIN B8 | 0.10 % |
| INCI: Biotin | |

Brij, Arlamol, Pricerine and Estol are registered trademarks of Uniqema; Glucam is a registered trademark of Union Carbide c/o Amerchol; Phenonip & Nipaguard are registered trademarks of NIPA Laboratories; Carbopol is a registered trademark of BF Goodrich; Myritol and Lanette are registered trademarks of Cognis; Dow Corning is a registered trademark of Dow Chemical. Folic acid was obtained from E.Merck, Darmstadt, Germany.

### PROCEDURE:

1. To prepare phase A, BRIJ 721 is mixed with water and heated to 75-80°C. A low viscous semi-transparent solution will be formed. The solution is cooled to 60°C. Propylene glycol and Glucam E20 are added and mixed until homogeneous. Phenonip and Nipaguard DCB are added.
2. To prepare phase B, Rhodicare D is dispersed in water, and subsequently Carbopol ETD2020 and Carbopol 2050 are dispersed. The dispersion is heated to 60°C while stirring gently.
3. Phase B is added to phase A and stirred until homogeneous. The temperature is maintained at 60°C.
4. Phase C is prepared by heating the mixture of ingredients to 60°C while mixing gently. Phase C is added to phase {A+B} while stirring gently. The mixture is homogenized during 20 seconds and the emulsion is subsequently stirred for 5 minutes, and phase D is added. The temperature of the emulsion is maintained at 60°C while stirring during 30 minutes.
5. The sodium hydroxide solution (phase E) is added to neutralize the acrylic resins present.
6. The emulsion is cooled to 45°C and phase F is added while stirring gently.
7. The emulsion is cooled to ambient temperature while mixing gently.
8. The emulsion is monitored for the absence of crystallites of folic acid.

### PRODUCT EVALUATION:

The emulsion obtained has a viscosity of 10.300 cPs Brookfield viscosity (S5; 20 rpm; 20°C) and a pH of 5,80. The emulsion is evaluated microscopically for the absence of crystallites and the occurrence of Maltese crosses. If crystallites are present, step 4 of the procedure has not been performed up to the requirements. If no or few Maltese crosses are observed, the liquid crystalline phase has not been formed.

### EXAMPLE 3

Treatment of individuals with a history of overexposure to UV irradiation using an O/W emulsion containing folic acid.

Two women of 84 and 51 years old respectively with a history of overexposure to UV irradiation and with small, superficial, slightly hardened and slightly discoloured spots on the skin of their underarms were asked to use a product according to the formula as provided in Example 1. Typical application frequency of the product was on a once-daily basis and involved approx 1 to 2 grams of product per arm per day.
After approx 4 weeks of application, the underarms were examined: with both women the tactility as well as colour of the spots was found to be reduced and much more in line with the touch and complexion of the surrounding, normal skin.

### EXAMPLE 4

Treatment of an individual with a history of exposure to chemical irritants using an O/W emulsion containing folic acid in the liquid crystalline matrix and vitamin B8 and provitamin B5 in the aqueous phase.

A male person of 48 years old with a hyperkeratotic area on one of his hands resulting from the handling of benzedine powder for a limited period of time some 30 years ago, was asked to use an emulsion according to the formula as provided in Example 2. Application of the skin care product on the hyperkeratotic area was on a once-daily basis and involved approx 0.5 grams of product per day.
After approx 3 weeks of application, the thickness of the hyperkeratotic area was found to be significantly reduced. Recurrence of the hyperkeratocity after stopping the product applications was not observed.

### EXAMPLE 5

Treatment of an individual with an outgrowth of verruceous tissue using an O/W emulsion incorporating folic acid.

A male person of 52 years old with a small verrucous outgrowth on his face was asked to use an emulsion according to the formula provided in Example 1. Application of the skin care product on the verruceous area was on a once-daily basis and involved approx 0.3 grams of product per day.
After approx 3 weeks of application, the size of the outgrowth was found to be significantly reduced. After termination of the treatment the size of the outgrowth hardly increased.

## Claims

1. An oil-in-water emulsion comprising substantially dissolved folic acid.

2. An oil-in-water emulsion comprising substantially dissolved folic acid as well as large numbers of liquid crystalline entities.

3. An emulsion according to claim 1 or 2 comprising an alkyl glucoside.

4. An emulsion according to claim 1 or 2 or 3 which further comprises at least one vitamin belonging to the group of B-vitamins other than folic acid.

5. A personal care product containing the emulsion according to claim 1 or 2 or 3 or 4.

6. The use of an emulsion according to claim 1 or 2 or 3 or 4 or a personal care product according to claim 5 to treat tactility or visibility of superficial skin blemishes or to treat tactility or visibility of hyperkeratotic skin.

7. The use of folic acid to to treat tactility or visibility of superficial skin blemishes or to treat tactility or visibility of hyperkeratotic skin.

8. The process for the production of an emulsion according to claim 1 or 2 or 3 or 4.

9. The process for the production of a personal care product according to claim 5.
